# EUROPEAN PATENT APPLICATION

(11) **EP 0 611 523 A1**
(43) Date of publication of application: **24.08.1994**
(21) Application number: 93908086.7
(22) Date of filing: 14.04.1993
(51) Int. Cl.: A01N 63/00, C12N 1/00

(54) **PROCESS FOR PRODUCING MICROBIAL NATURAL ENEMY OF NEMATODE BY UTILIZING WHOLE PLANT BODY**

(30) Priority: 14.04.1992 JP 119528/92
(71) Applicant: NEMATECH CO., LTD., Minato-ku, Tokyo (JP)
(72) Inventor: IKEDA, Ruriko, Nematech Co. Ltd., Ibaragi-ken 305 (JP); KAWADA, Hiroshi, Nematech Co. Ltd., Ibaragi-ken 305 (JP); KASUMIMOTO, Takanori, Nematech Co. Ltd., Ibaragi-ken 305 (JP)
(74) Representative: Kraus, Walter, Dr.
(86) International application number: JP9300474
(87) International publication number: WO9320697

(57) **Abstract**

A process for producing a microbial natural enemy of nematodes by utilizing a whole plant body, which comprises making plant parasitic nematodes bearing the adherent microbial natural enemy invade a whole plant body, thereby efficiently producing an obligate parasite acting as a natural enemy of nematodes in the whole plant body.

## Description

### Technical Field

This invention relates to a method for producing an absolute parasitic microorganism which is natural enemy to plant parasitic nematode which brings about damages to crop plants.

### Background of Technology

To avoid damages caused by nematode, it has been common to employ a chemical control method or a field husbandly control method. From an economical background such that profitable crop plants have to be cultivated by continuous cropping, a convenient inexpensive chemical control method has been widely used. However, in view of a concern about environmental pollution due to treatment with large amounts of a chemical agent, a study is also being made on a method of employing a natural enemy microorganism as one of new control methods

An attention has been drawn particularly to Pasteuria spp. i.e. a group of bacteria which are absolute parasites to nematode, since they are parasitic specifically to certain specific nematodes and have relatively high resistance against heat and chemical substances and thus can be stored for a long period of time, and thus they are highly safe and easy to use. However, it has been extremely difficult to parasitize them efficiently on nematode and multiply them in a large amount, because they are absolute parasites and have no motility. For multiplication of the microorganism of this type it has been common to employ a method wherein nematode parasitized with such a microorganism is inoculated in the vicinity of the soil on which a crop plant is cultivated, followed by cultivation for a long period of time of at least two months, whereupon the multiplied microorganism is recovered from the root. However, the method is low in productivity, and is thus hardly practicable for actual agricultural production. In addition, it is possible that a plant root transformed by agrobacterium or a simple root system is aseptically cultured on a culture medium such as an agar medium, and then nematode parasitized with the microorganism is aseptically inoculated thereto and multiplied under a controlled environment. However, the productivity is not better than the method using the soil.

Furthermore, multiplication of plant parasitic nematode in the past has been conducted only with the root, and naturally the bacterium parasitic to nematode has been multiplied only using the plant root.

In general, penetration of nematode into a plant is limited in the vicinity of the growth point of root, and thus can limitedly be controlled by the cultivation area, plant type, or the like. Furthermore, the number of nematode population per plant must be limited in order to maintain the crop plant because substantial damages to the root due to inoculation of large amounts of nematode, and it has thus been difficult to improve the productivity.

The inventors have found that not only the root which has been used as a production location, but also the stem or the leaves can be used which are easily penetrated with nematode parasitized with the microorganism to produce the microorganism. Specifically, nematode parasitized with the microorganism is dispersed in an aqueous solution of polysaccharide or its derivative, and the entire plant is immersed in the solution. Alternatively, the nematode dispersion is sprayed onto the plant, or the entire plant is embedded in an inorganic or organic carrier which is lighter than water, on which nematode parasitized with the microorganism is inoculated. Thus, nematode parasitized with the microorganism is penetrated to the stem or leaves by the above methods.

It has been found that the objective natural enemy microorganism can be obtained in a large amount over the prior art methods by cultivation under an appropriate temperature control, thus accomplishing the present invention.

### Disclosure of the Invention

In accordance with the present invention, there is provided a method for producing a microorganism as a natural enemy to nematode comprising inoculating the natural enemy microorganism-deposited plant parasitic nematode on the surface of the entire plant including its stem and leaves. Specifically, the plant parasitic nematode parasitized with the natural enemy microorganism is dispersed in a solution of polysaccharide or its derivative and the entire plant is immersed in the solution, or the solution is sprayed on the entire plant to penetrate the nematode into the plant, or the entire plant is embedded in an inorganic or organic carrier which is lighter than water, the natural enemy microorganism-deposited nematode dispersed in water is added from the top for penetrating the nematode into the entire plant, and the carrier is removed, thereby producing the natural enemy microorganism.

The nematode useful in the present invention is preferably a root knot nematode or a cyst nematode.

The plant may be any plant as long as such a nematode can be parasitic thereon.

The polysaccharide and its derivative include agar, geran gum, carboxymethylcellulose, and the like. The inorganic carrier can be microballoons, which are cinders of coal or coke, and the like, and the organic carrier can be pulverized rice hull, peat moss, and the like.

### Best Mode for Practicing the Invention

The present invention will now be described further in detail with reference to Examples.

### Example 1

### Penetration of nematode into stem and leaves

Seeds were sown in microballoons, and when twin leaves appeared after cultivation for ten days, nematodes having natural enemy microorganism Pasteuria spp. deposited thereon were applied. Specifically, sweet potato root knot nematodes deposited with the natural enemy microorganism were added to 0.2% aqueous agar solution so that 600 nematodes were contained per ml, and the plant removed from the microballoons was immersed in the solution. The result was put in a polyethylene bag and allowed to stand at room temperature. Or, the solution was sprayed onto the entire plant, which was put in the polyethylene bag and allowed to stand at room temperature.

Furthermore, microballoons were put on the plant cultivated on the microballoons until the plant is embedded in the microballoons, and the aqueous dispersion of the nematodes deposited with the natural enemy microorganism was put thereon in an amount of 100 nematodes per plant. After that, a sufficient amount of water was supplied to the plant to disperse the nematodes in the soil. After 2 days from the application, the plant was washed with 15% antiformin for 5 minutes and then with water, immersed in an acidic Fuchsine solution and boiled. When cooled to an appropriate temperature, it was decolorized with acidic glycerol. The number of nematodes penetrated into the stem or leaves of each sample was counted. The results are shown in Table 1.

**Table 1**

| | Agar culture | | Agar spray | | Microballoon | |
|---|---|---|---|---|---|---|
| | Stem | Leaf | Stem | Leaf | Stem | Leaf |
| Tomato | 8 | 1 | 3 | 1 | 16 | 16 |
| Eggplant | 14 | 16 | 4 | 6 | 28 | 41 |
| Tobacco | 6 | 1 | 4 | 0 | 10 | 0 |
| Cucumber | 5 | 2 | 5 | 1 | 12 | 4 |
| White rape | 3 | 10 | 4 | 6 | 4 | 19 |
| Lettuce | 7 | 0 | 6 | 0 | 10 | 0 |

### Example 2

### Production of the natural enemy microorganism utilizing stem and leaves

Seedlings of tomato, eggplant, and potato were cultivated by means of black volcanic ash soil filled to a half of a 1/10,000a pot. When the plants had stems and leaves of about 15 cm, microballoons were added to the pot, about 10,000 nematodes deposited with the natural enemy microorganism (Pasteuria sp.) were inoculated to each plant, and then sufficient amounts of water were supplied. After 5 days, a large amount of water was supplied to the pot to remove the microballoons utilizing the difference in specific gravity. Cultivation was conducted at 25°C for 8 weeks. After the cultivation, the root, and the stem and leaves were separately collected and homogenized, the homogenized solution was filtered out, and the produced natural enemy microorganisims were counted on a microscope. The results are shown in Table 2.

**Table 2**

| | Number of produced microorganisms per seedling | |
|---|---|---|
| | Root | Stem, leaf |
| Tomato | 3.4 x 10⁸ | 1.9 x 10⁸ |
| Eggplant | 2.4 x 10⁸ | 8.4 x 10⁷ |
| Potato | 8.3 x 10⁷ | 2.7 x 10⁷ |

### Utilizability in Industry

With the present invention, it is made possible to efficiently produce the absolute parasitic microorganism which is natural enemy to plant parasitic nematode, which was difficult to be produced in the past.

## Claims

1. A method for producing a microorganism which is natural enemy to nematode, comprising penetrating plant parasitic nematode deposited with the natural enemy microorganism into the entire plant including the stem and leaves.

2. A method for producing a microorganism which is natural enemy to nematode, comprising dispersing plant parasitic nematode deposited with the natural enemy microorganism in a solution of a polysaccharide and a derivative thereof and immersing the entire plant into the solution, or spraying the solution on the entire plant, whereby penetrating the nematode into the entire plant.

3. A method for producing a microorganism which is natural enemy to nematode, comprising embedding the entire plant in an inorganic or organic carrier which is lighter than water, dispersing the nematode deposited with the natural enemy microorganism, adding the dispersion to the carrier to penetrate the nematode into the entire plant, and removing the carrier.
